# EUROPEAN PATENT SPECIFICATION

(11) **EP 2 638 088 B1**
(45) Date of publication and mention of the grant of the patent: **18.11.2015**
(21) Application number: 11763843.7
(22) Date of filing: 02.09.2011
(51) Int. Cl.: C08G 18/10, C08G 18/32, C08G 18/38, C07C 209/68, C07C 209/74, C07C 211/51, C07C 213/00, C07C 217/80, C08G 59/50

(54) **HALOGENATION OF 4,4'-METHYLENE-BIS-ANILINES**
HALOGENIERUNG VON 4,4'-METHYLEN-BIS-ANILINEN
HALOGÉNATION DES 4,4'-MÉTHYLÈNE-BIS-ANILINES

(30) Priority: 02.09.2010 EP 10175021
(43) Date of publication of application: 18.09.2013
(73) Proprietor: Lonza Ltd, 3930 Visp (CH)
(72) Inventor: ABGOTTSPON, Magnus, CH-3933 Staldenried (CH); ELLINGER, Stefan, CH-3930 Visp (CH); LA DELFA, Gaetano, CH-3904 Naters (CH); FURRER, Martina, CH-3930 Visp (CH); FRANZKE, Constanze, 67063 Ludwigshafen (DE); ZUR TÄSCHLER, Cornelia, CH-3930 Visp (CH)
(74) Representative: Riegler, Norbert Hermann
(86) International application number: PCT/EP2011/004423
(87) International publication number: WO 2012/028323

(56) References cited:
- WO-A1-97/21749
- GB-A- 1 044 509
- GB-A- 1 207 377
- US-A- 3 752 768
- US-A- 4 950 792

## Description

The present invention is directed to a process for the preparation of halogenated 4,4'-methylene-bis-anilines.

The use of chain extenders and curing agents (in the following collectively called curing agents) for the preparation of polyurethanes and epoxy resins is well known in the art. Polyurethanes, for example, may be obtained by reacting compounds having H-reactive groups such as polyether polyols or polyester polyols with a diisocyanate to form a prepolymer, which in a second step is then reacted with a curing agent to form the polyurethane. Epoxy resins, on the other hand, may be obtained by reacting epichlorohydrin with an alcohol to obtain a glycidyl derivative which then is reacted with a curing agent to obtain the cured epoxy resin.

The structure of the curing agents and the reactivity of their functional groups are often used to modify the properties of the final product or to control the reaction rate of the polymer formation and the processability of the polymer.

Commonly used curing agents for the preparation of polyurethanes (PU) and epoxy resins are chlorinated 4,4'-methylene-bis-anilines. The effect of such compounds on the properties of the polymer system substantially depends on the alkyl substituents and/or the number and/or positions of the chlorine atoms on the aromatic rings. Sterically hindered diamines such as 4,4'-methylenebis-(3-chloro-2,6-diethylaniline) (M-CDEA) are often used as a curing agent to adjust gel times. These compounds, however, do not allow to flexibly control gel times over a broad range. Other well known compounds such as 4,4'-methylenebis-(o-chloroaniline) (MOCA) tend to be toxic.

Chlorinated 4,4'-methylene-bis-anilines are conventionally prepared by subjecting a chlorinated aniline to a condensation reaction with formaldehyde.

EP-A-0 171 588 discloses the preparation of chlorinated 4,4'-methylene-bis-anilines by subjecting 2-ethyl-6-chloroaniline and various 2,6-dialkylanilines to a condensation reaction with formaldehyde. The condensation reaction, however, is time consuming and often requires a high energy input. Moreover, following condensation the reaction product contains unreacted starting materials which must be removed before use as it results in endcapping of the formed polymer. While mixtures of chlorinated aromatic diamines obtained by this method can be used to vary the pot lives of polyurethane polymers, the directed preparation of specific mixtures of chlorinated 4,4'-methylene-bis-anilines is difficult. Moreover, methylene-bis-anilines having chlorine atoms in ortho-position to the amino group tend to be toxic.

EP-A-0 220 641 describes a method for preparing meta-chlorinated 4,4'-methylene-bis(2,6-dialkylanilines) by subjecting meta-chlorinated 2,6-dialkylanilines as a starting material to a condensation reaction with formaldehyde. This method, therefore, suffers from the same disadvantages as described above.

The object of the present invention, therefore, is to provide an effective and cost efficient process for the preparation of meta-halogenated 4,4'-methylene-bis-anilines and mixtures thereof.

According to the invention, this has been achieved by a process for the preparation of halogenated aromatic amines of formula (1) 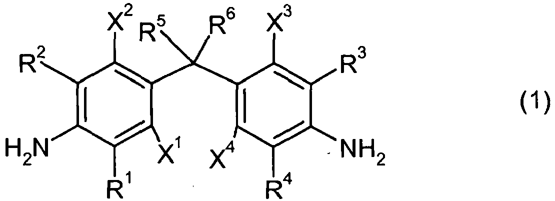 wherein R¹, R², R³ and R⁴ independently represent H, Cl or Br, an alkyl group or an alkoxy group, R⁵ and R⁶ independently represent H or an alkyl group or together with the carbon atom to which they are bound form a cyclopentane ring or a cyclohexane ring, and X¹, X², X³ and X⁴ independently represent H, Cl or Br, wherein at least one of X¹, X², X³ and X⁴ is Cl or Br, or mixtures thereof,
said process comprising:
(a) providing an aromatic amine of the formula (2) 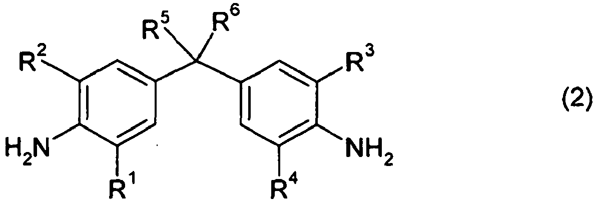 wherein R¹, R², R³, R⁴, R⁵ and R⁶ are as defined above, in the anilinium form in a mixture with sulfuric acid; and
(b) treating the mixture of step (a) with chlorine (Cl₂) or bromine (Br₂), followed by treatment with a base to obtain the halogenated aromatic amine of the formula (1).

It has been found that the process of the invention allows selective meta-halogenation of 4,4'-methylene-bis-anilines in high yields without undesired side products.

The term alkyl as used in the definitions of R¹, R², R³, R⁴, R⁵ and R⁶ includes linear and branched alkyl groups. Preferably, the alkyl group is a C₁₋₆ alkyl group, more preferably a C₁₋₄ alkyl group. Preferred alkyl groups include methyl, ethyl, *n*-propyl, isopropyl, *n*-butyl, *sec*-butyl and *tert*-butyl. More preferred alkyl groups are methyl, ethyl, *n*-propyl, isopropyl and *sec*-butyl. Most preferred alkyl groups are methyl, ethyl, *n*-propyl and isopropyl.

The term alkoxy as used in the definitions of R¹, R², R³, R⁴, R⁵ and R⁶ includes linear and branched alkoxy groups. Preferably, the alkoxy group is a C₁₋₆ alkoxy group, more preferably a C₁₋₄ alkoxy group. Preferred alkoxy groups include methoxy, ethoxy, *n*-propoxy, isopropoxy, *n*-butoxy, *sec*-butoxy and *tert-*butoxy. More preferred alkoxy groups are methoxy, ethoxy, *n*-propoxy, isopropoxy and *n*-butoxy. Most preferred alkoxy groups are methoxy, ethoxy, *n*-propoxy and isopropoxy.

According to a preferred embodiment of the process of the invention, at least one of R¹ and R² and at least one of R³ and R⁴ are different from H.

Preferably, R¹, R², R³ and R⁴ independently represent H or an alkyl group and R⁵ and R⁶ represent H or together with the carbon atom to which they are bound form a cyclohexane ring. More preferably, at least one of R¹, R², R³ and R⁴ is an alkyl group, i. e., at least one of the two aromatic rings of the compounds of formulas (1) and (2) carries at least one alkyl group in ortho-position to the amino group. Even more preferably, at least one of R¹ and R² and at least one of R³ and R⁴ is an alkyl group, i. e., each of the two aromatic rings of the compounds of formulas (1) and (2) carries at least one alkyl group in ortho-position to the amino group, wherein these alkyl groups preferably are identical.

According to a further preferred embodiment of the invention, all of R¹, R², R³ and R⁴ independently represent an alkyl group. In this case, the alkyl groups of R¹ and R² preferably correspond to the alkyl groups R³ and R⁴, i. e., the alkyl groups of the aromatic rings of the compounds of the formula (1) and (2) are symmetrically arranged. According to a particular preferred embodiment of the invention, the alkyl groups represented by R¹, R², R³ and R⁴ are the same.

The number of halogen atoms represented by X¹, X², X³ and X⁴ in the compounds of the formula (1) may vary from 1 to 4. Preferably, if X¹ and X² both are Cl or Br, at least one of X³ and X⁴ is Cl or Br, respectively, and vice versa. Preferably, X¹, X², X³ and X⁴ independently represent H or Cl.

The sulfuric acid (H₂SO₄) used in the present process in admixture with the aromatic amines of the formula (2) preferably is a sulfuric acid having an acid concentration of at least 70% by weight, more preferably of at least 85% by weight, even more preferably of at least 90% by weight, and most preferably of at least 95% by weight. While halogenation can be carried out using a sulfuric acid having an acid concentration of below 70% by weight, yield is better at higher concentrations.

Typically, the aromatic diamine of the formula (2) is provided in the anilinium form by mixing the amino compound of the formula (2) with sulfuric acid. Following mixing of the two components, the amine will essentially be present in the form of a sulfuric acid salt, i.e., an anilinium salt, which reacts with the chlorine or bromine. Advantageously, the sulfuric acid is used in a molar excess with respect to the aromatic diamine. This allows better mixing and probably dissolution of the formed anilinium salt in the sulfuric acid. For improved yields, thus, the molar ratio of sulfuric acid to amino compound of the formula (2) in the starting mixture preferably should be at least 2:1, more preferably at least 5:1 and most preferably at least 10:1. Molar ratios of sulfuric acid to amino compound of 40:1 and more will be possible but will not improve yield. Alternatively, the compound of the formula (2) can be provided in the form of an anilinium salt, preferably a sulfuric acid salt such as an anilinium hydrogensulfate or an anilinium sulfate, which is mixed with the sulfuric acid. Preferably, to optimize yields, the mixture to be treated with the chlorine or bromine essentially consists of the amine of the formula (2) in the anilinium form and of the sulfuric acid.

Halogenation of the compound of the formula (2) is typically effected using gaseous chlorine (Cl₂) or liquid bromine (Br₂) which are introduced into or passed through the mixture with the sulfuric acid. Preferably, the process of the invention is carried out using chlorine. The reaction is typically carried out at a temperature in a range of from 5 °C to 150 °C, preferably of from 5 °C to 120 °C, and more preferably of from 5 °C to 100 °C. Lower or higher temperatures may work as well, but lower temperatures may result in a decreased reaction rate while higher temperatures do not increase yield.

The halogenation reaction is followed by treatment with a base to neutralize the sulfuric acid and to liberate the chlorinated or brominated aniline of the formula (1) from the sulfuric acid salts which are formed as intermediates. Bases which are typically used for the treatment include organic and inorganic bases such as triethylamine and alkali metal hydroxides and carbonates. Sodium or potassium hydroxide are preferred and are typically used in the form of aqueous solutions.

The liberated halogenated aromatic diamine of the formula (1) can be isolated according to known methods, for example by separating the organic layer or by extraction with a water immiscible solvent such as dichloromethane, ethyl acetate or toluene. The solvent may then be removed under reduced pressure. The halogenated aniline compounds may be purified by distillation.

Thus, the process for the preparation of halogenated aromatic amines of the formula (1) according to the present invention may further comprise the step of purifying the halogenated aromatic amine of the formula (1) obtained in above step (b).

The process of the invention may be carried out in a closed or in an open system.

According to a preferred embodiment of the invention, the halogenation reaction is carried out in a closed system, for example a sealed reaction vessel such as a sealed autoclave. A closed system has the advantage that it does not exchange matter with its surroundings during the reaction and thus allows easy control of the stoichiometry of the reactants. Typically, in the process of the invention the mixture of amino compound and sulfuric acid is charged into the reaction vessel, preferably an autoclave, and the reaction vessel is then sealed and the calculated amount of halogen is added. Due to the closed system, none of the reactants will leak so that a predetermined molar ratio of the reactants can be provided for controlling the process.

Alternatively, the halogenation reaction of the invention can be carried out in an open system which allows the flow of matter out of the system boundaries. The open system used in the process of the invention may be an open reaction vessel, for example a standard glass apparatus such as a three-necked round bottom flask, which is charged with the amino compound and the sulfuric acid before the respective halogen is added. Conveniently, if chlorine is used, the chlorine gas is passed through the reaction mixture, for example using a dip pipe.

The degree of halogenation may be controlled, for example, by adjusting the stoichiometry of the reactants, that is, the molar ratio of halogen to aromatic diamine of the formula (2). The optimum molar ratio for the desired degree of halogenation may be determined by routine experiments by the person skilled in the art.

In case of a chlorination reaction, for example, which is carried out in a closed reaction vessel, a mono-chlorinated product is obtained in high yields if chlorine (Cl₂) and starting material of the formula (2) are used in about stoichiometric amounts, i.e., if the molar ratio of chlorine molecules (Cl₂) to starting material is in the range of from about 0.90:1 to 1.10:1, more preferably in the range of from about 0.95:1 to 1.05:1.

Under the same conditions, if the molar ratio of chlorine molecules (Cl₂) to starting material is increased to from about 1.90:1 to 2.30:1, more preferably to from about 1.95:1 to 2.2:1, the di-chlorinated product wherein one of X¹ and X² and one of X³ and X⁴ is Cl is obtained as the main product and in high yields.

Under the same conditions, if the molar ratio of chlorine molecules (Cl₂) to starting material is increased to from about 2.90:1 to 3.50:1, more preferably to from about 2.95:1 to 3.3:1, a tri-chlorinated product is obtained as the main product and in high yields.

The tetra-chlorinated product can be obtained as the main product and in high yields if the molar ratio of chlorine molecules (Cl₂) to starting material is adjusted to above about 3.9:1, preferably to above about 5.0:1.

If the chlorination reaction is carried .out in an open reaction vessel, the mono- and di-chlorinated 4,4'-methylene-bis-anilines are obtained in high yields if chlorine is passed through the reaction mixture. The higher chlorinated compounds will be obtained only upon longer reaction times and by passing large amounts of chlorine through the reaction mixture.

The same molar ratios may be used in case of bromination. Higher concentrations of bromine, however, may be advantageous.

The process of the present invention allows a controlled halogenation of 4,4'-methylene-bis-anilines of the formula (2) in high yields and with few side products. Unreacted starting material can be recycled. The present process, thus, can be carried out in a simple manner and at low costs.

Further advantages of the present invention will be obvious from the following description of specific non-limiting examples which illustrate the present invention.

### Examples

The following examples illustrate the chlorination of 4,4'-methylene-bis-(dialkylanilines) in the presence of sulfuric acid according to the process of the invention using a sealed autoclave to allow precise control of the stoichiometry of the chlorination reaction. All examples use sulfuric acid having a concentration of 96% by weight. Gas chromatography (GC) was carried out using an Agilent 6850 apparatus and a dimethylpolysiloxane-coated capillary as the separation column (30 m × 0.32 mm × 0.35 µm).

### Example 1

11.8 g (38.0 mmol) of 4,4'-methylene-bis-(2,6-diethylaniline) and 58.6 g (574 mmol) of sulfuric acid (96% by weight) were introduced into an autoclave made of Hastelloy^{®} HC22. The autoclave was cooled to 15 °C and flushed with nitrogen to check leak-tightness. Following release of the nitrogen, 2.7 g (38.1 mmol) of chlorine gas were introduced into the mixture. The reaction was stirred at 15 °C for 1 h and then poured into 50 g of ice. The suspension was neutralized with 440 g of 10% aqueous sodium hydroxide and then extracted with 210 g of toluene. Following phase separation, the organic layer was dried over anhydrous magnesium sulfate and evaporated under reduced pressure to give 14.7 g of a crude product. According to gas chromatography the crude product contained 55.3% of 3-chloro-4,4'-methylenebis-(2,6-diethylaniline), 25.8% of 4,4'-methylenebis-(3-chloro-2,6-diethylaniline) and 18.2% of unreacted 4,4'-methylenebis-(2,6-diethylaniline).

### Example 2

Example 1 was repeated with a reaction temperature of 30 °C and a reaction time of 3 h. The crude product obtained contained (according to GC) 54.2% of 3-chloro-4,4'-methylene-bis-(2,6-diethylaniline), 27.0% of 4,4'-methylenebis-(3-chloro-2,6-diethylaniline) and 15.4% of unreacted 4,4'-methylenebis-(2,6-diethylaniline).

### Example 3

10.0 g (32.2 mmol) of 4,4'-methylenebis-(2,6-diethylaniline) and 48.8 g (478 mmol) of sulfuric acid (96% by weight) were introduced into an autoclave made of Hastelloy^{®} HC22. The autoclave was heated to 30 °C and flushed with nitrogen to check leak-tightness. Following release of the nitrogen, 4.5 g (63.5 mmol) of chlorine gas were introduced into the mixture. The reaction was stirred at 30 °C for 4.5 h and then poured into 50 g of ice. The suspension was neutralized with 420 g of 10% aqueous sodium hydroxide and then extracted with 200 g of toluene. Following phase separation, the organic layer was dried over anhydrous magnesium sulfate and evaporated under reduced pressure to give 12.0 g of a crude product containing (according to GC) 91.7% of 4,4'-methylenebis-(3-chloro-2,6-diethylaniline) and 5.5% of 3-chloro-4,4'-methylenebis-(2,6-diethylaniline).
[C₂₁H₂₈Cl₂N₂]:
¹H NMR (CDCl₃, 400 MHz): δ: 1.14 (t, *J* = 7.5 Hz, 6H, CH₃), 1.18 (t, *J* = 7.5 Hz, 6H, CH₃), 2.42 (q, *J* = 7.5 Hz, 4H, CH₂), 2.79 (q, *J* = 7.5 Hz, 4H, CH₂), 3.64 (br s, 4H, NH₂), 4.03 (br s, 2H, CH₂), 6.61 (s, 2H, 5-Ar-H).
GC (dimethylpolysiloxane, 30 m × 0.32 mm × 0.35 µm; 90 °C, 30 K/min → 200 °C, 20 K/min → 260 °C, 2 K/min → 280 °C): *t*_{R} = 13.4 min.

### Example 4

Example 2 was repeated with a molar ratio of chlorine to 4,4'-methylenebis-(2,6-diethylaniline) of 3.5: 1. The crude product obtained contained (according to GC) 51.3% of 3,3',5-trichloro-4,4'-methylenebis-(2,6-diethylaniline), 32.9% of 4,4'-methylenebis-(3,5-dichloro-2,6-diethylaniline) and 15.9% of 4,4'-methylenebis-(3-chloro-2,6-diethylaniline).

### Example 5

7.2 g (23.2 mmol) of 4,4'-methylenebis-(2,6-diethylaniline) and 70.2 g (687 mmol), of sulfuric acid (96% by weight) were introduced into an autoclave made of Hastelloy^{®} HC22. The autoclave was heated to 30 °C and flushed with nitrogen to check leak-tightness. Following release of the nitrogen, 12.3 g (173.5 mmol) of chlorine gas were introduced into the mixture. The reaction was stirred at 30 °C for 3 h and then poured into 100 g of ice. The suspension was neutralized with 660 g of 10% aqueous sodium hydroxide and then extracted with 400 g of dichloromethane. Following phase separation, the organic layer was dried over anhydrous magnesium sulfate and evaporated under reduced pressure to give 9.6 g of a crude product containing (according to GC) 99.1% of 4,4'-methylenebis-(3,5-dichloro-2,6-diethylaniline). [C₂₁H₂₆Cl₄N₂]:
¹H NMR ((CD₃)₃SO), 400 MHz): δ: 1.24 (t, *J* = 7.5 Hz, 12H, CH₃), 2.86 (q, *J* = 7.5 Hz, 8H, CH₂), 3.91 (br s, 4H, NH₂), 4.72 (br s, 2H, CH₂).
GC (dimethylpolysiloxane, 30 m × 0.32 mm × 0.35 µm; 90 °C, 30 K/min → 200 °C, 20 K/min → 260 °C, 2 K/min → 280 °C): *t*_{R} = 18.5 min.

The results of examples 1 to 5 are summarized in Table 1, wherein the terms M-DEA, M-DEA-Cl, M-CDEA, M-DEA-Cl3 und M-DEA-Cl4 represent the following compounds:

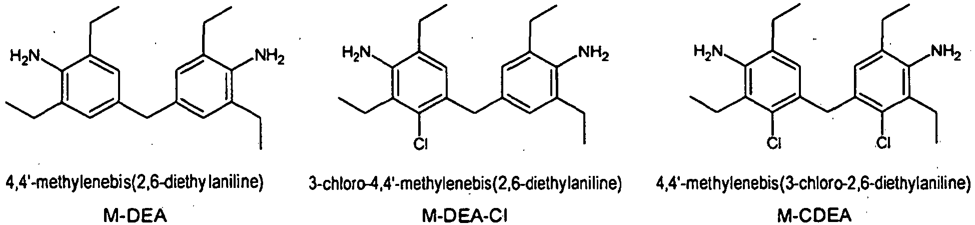

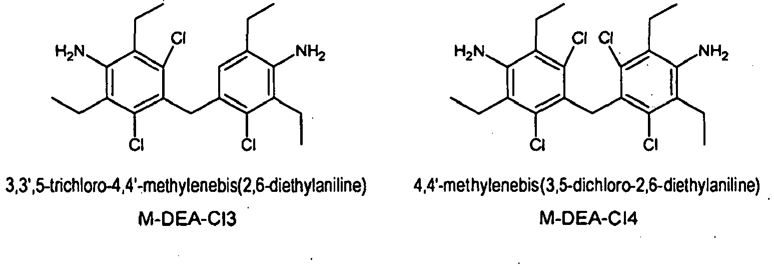

**Table 1**

| Example | Molar ratio Cl₂:M-DEA | Reaction temperature (°C) | Time (h) | Proportion in product mixture⁽¹⁾ | | | | |
|---|---|---|---|---|---|---|---|---|
| | | | | M-DEA | M-DEA-Cl | M-CDEA | M-DEA-Cl3 | M-DEA-Cl4 |
| 1 | 1:1 | 15 | 1 | 18.2% | 55.3% | 25.8% | 0.0% | 0.0% |
| 2 | 1:1 | 30 | 3 | 15.4% | 54.2% | 27.0% | 0.0% | 0.0% |
| 3 | 2:1 | 30 | 4.5 | 0.3% | 5.5% | 91.7% | 1.2% | 0.0% |
| 4 | 3.5:1 | 30 | 3 | 0.0% | 0.0% | 15.9% | 51.3% | 32.9% |
| 5 | 7.5:1 | 30 | 3 | 0.0% | 0.0% | 0.0% | 0.00% | 99.1% |

| | | | | | | | | |
|---|---|---|---|---|---|---|---|---|
| ⁽¹⁾ The proportions of the compounds in the product mixture are calculated on the basis of the areas of the respective peaks in a gas chromatogram. | | | | | | | | |

Examples 1 to 5 demonstrate that by controlling the stoichiometry of the reaction, i. e. the molar ratio of chlorine (Cl₂) to M-DEA, high yields of chlorinated methylene-bis-anilines having a specific grade of chlorination could be obtained. A comparison of examples 1 and 2 further shows that the same selectivities and yields were obtained even at a reduced reaction temperature of 15 °C and a reduced reaction time of 1 h.

### Example 6

Example 3 was repeated under identical conditions except that 4,4'-methylene-bis(2,6-diisopropylaniline) was used as the starting material. After a reaction time of 3 h and usual work up a crude product was obtained containing a mixture of products of the chlorinated starting material with 4,4'-methylenebis-(3-chloro-2,6-diisopropyl-aniline) being the major product with a content of 73.0% (according to GC).

GC (dimethylpolysiloxane, 30 m × 0.32 mm × 0.35 µm; 100 °C, 30 K/min → 300 °C): *t*_{R} = 7.6 min.

### Example 7

Example 5 was repeated under identical conditions except that 4,4'-methylene-bis-(2,6-diisopropylaniline) was used as the starting material. After a reaction time of 4 h and usual work up a crude product was obtained containing a mixture of products of the chlorinated starting material with 4,4'-methylenebis-(3,5-dichloro-2,6-diisopropyl-aniline) being the major product with a content of 72.7% (according to GC).

GC (dimethylpolysiloxane, 30 m × 0.32 mm × 0.35 µm; 100 °C, 30 K/min →300° C): *t*_{R} = 8.9 min.

The results of examples 6 and 7 are summarized in Table 2, wherein the terms M-DIPA, M-DIPA-Cl, M-DIPA-Cl2, M-DIPA-Cl3 and M-DIPA-Cl4 represent the following compounds:

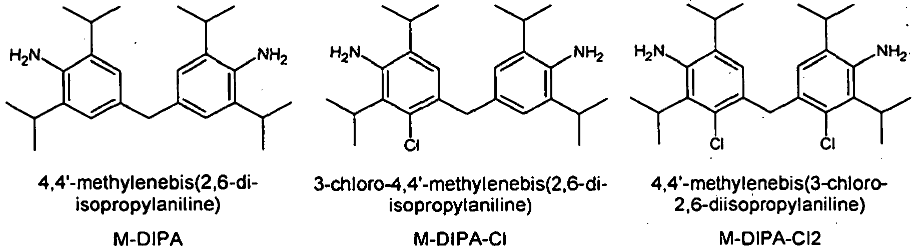

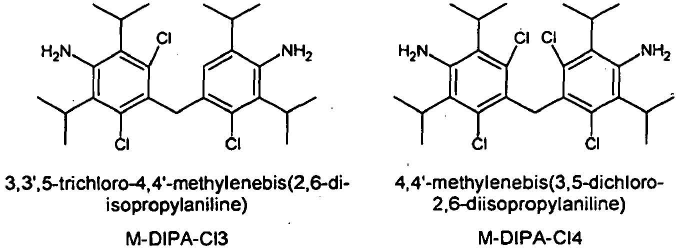

**Table 2**

| Example | Molar ratio Cl₂:M-DIPA | Reaction temperature (°C) | Time (h) | Proportion in product mixture⁽¹⁾ | | | | |
|---|---|---|---|---|---|---|---|---|
| | | | | M-DIPA | M-DIPA-Cl | M-DIPA-Cl2 | M-DIPA-Cl3 | M-DIPA-Cl4 |
| 6 | 2:1 | 30 | 3 | 7.4% | 0.0% | 73.0% | 18.0% | 0.0% |
| 7 | 7.5:1 | 30 | 4 | 0.0% | 0.0% | 0.0% | 13.8% | 72.7% |

| | | | | | | | | |
|---|---|---|---|---|---|---|---|---|
| ⁽¹⁾ The proportions of the compounds in the product mixture are calculated on the basis of the areas of the respective peaks in a gas chromatogram. | | | | | | | | |

Similar to Examples 1 to 5 above, Examples 6 and 7 demonstrate that use of 4,4'-methylenebis-(2,6-diisopropylaniline) as the starting material also results in high yields of chlorinated methylene-bis-anilines having a specific grade of chlorination when controlling the stoichiometry of the reaction, i.e. the molar ratio of chlorine (Cl₂) to M-DIPA.

### Example 8

Example 3 was repeated under identical conditions except that 4,4'-methylene-bis-(2-isopropyl-6-methylaniline) was used as the starting material and the reaction time was 3 h. The crude product obtained was a mixture of products of the chlorinated starting material with 4,4'-methylenebis-(3-chloro-6-isopropyl-2-methylaniline) being the major product with a content of 80.0% (according to GC).

GC (dimethylpolysiloxane, 30 m × 0.32 mm × 0.35 µm; 70 °C, 25 K/min → 260 °C, 5 K/min → 270 °C, 25 K/min → 300 °C): *t*_{R} = 12.8 min.

### Example 9

Example 5 was repeated under identical conditions except that 4,4'-methylene-bis-(2-isopropyl-6-methylaniline) was used as the substrate and the molar ratio of chlorine (Cl₂) to starting material was 7:1. After a reaction time of 3 h, a crude product was obtained containing a mixture of products of the chlorinated starting material with 4,4'-methylene-bis-(3,5-dichloro-6-isopropyl-2-methylaniline) being the major product with a content of 81.9% (according to GC).
GC (dimethylpolysiloxane, 30 m × 0.32 mm × 0.35 µm; 70 °C, 25 K/min → 260 °C, 5 K/min → 270 °C, 25 K/min → 300 °C): *t*_{R} = 15.1 min.

The results of examples 8 and 9 are summarized in Table 3, wherein the terms M-MIPA, M-MIPA-Cl, M-MIPA-Cl2, M-MIPA-Cl3 and M-MIPA-Cl4 represent the following compounds with M-MIPA-Cl, M-MIPA-Cl2 and M-MIPA-Cl3 including the various isomers of the mono-, di- and trichloroanilines:

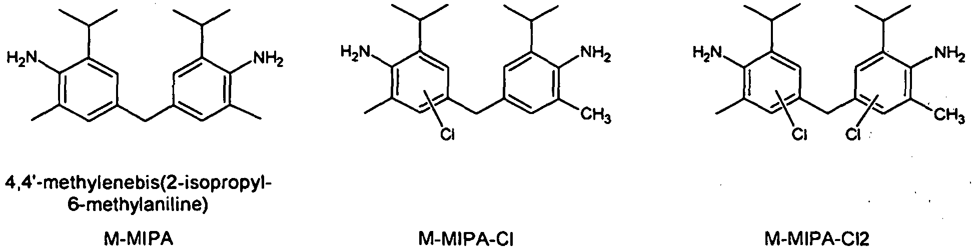

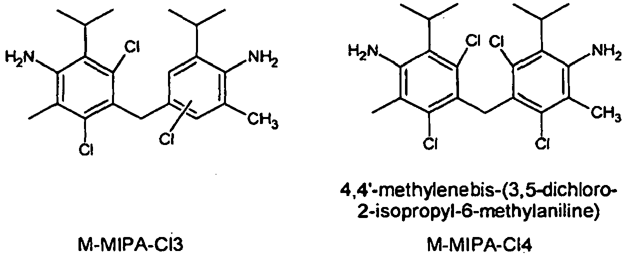

**Table 3**

| Example | Molar ratio Cl₂ : M-MIPA | Reaction temperature (°C) | Time (h) | Proportion in product mixture⁽¹⁾ | | | | |
|---|---|---|---|---|---|---|---|---|
| | | | | M-MIPA | M-MIPA-Cl | M-MIPA-Cl2 | M-MIPA-Cl3 | M-MIPA-Cl4 |
| 8 | 2:1 | 30 | 3 | 4.8% | 0.0% | 80.0% | 3.2% | 0.0% |
| 9 | 7:1 | 30 | 3 | 0.0% | 0.0% | 0.0% | 0.0% | 81.9% |

| | | | | | | | | |
|---|---|---|---|---|---|---|---|---|
| ⁽¹⁾ The proportions of the compounds in the product mixture are calculated on basis of the areas of the respective peaks in a gas chromatogram. | | | | | | | | |

Again, examples 8 and 9 demonstrate that by controlling the stoichiometry of the chlorination reaction high yields of chlorinated 4,4'-methylenebis-(2,6-dialkylanilines) having specific grade of chlorination can be achieved.

Examples 1 to 9 demonstrate that the process of the invention allows the preparation of product mixtures containing high proportions of a specific chlorinated methylene-bis-aniline. By modifying the reaction conditions such as reaction temperature, reaction time and, in particular, the molar ratio of Cl₂ to starting material (like M-DEA, M-DIPA and M-MIPA), however, it is possible to also obtain mixtures containing two or more different reaction products in considerable amounts.

## Claims

1. A process for the preparation of halogenated aromatic amines of the formula (1) 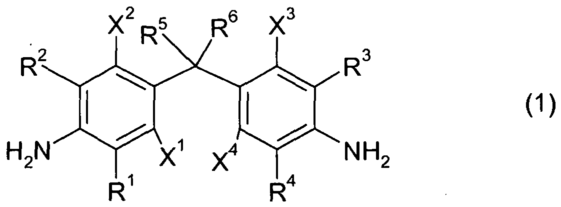 wherein R¹, R², R³ and R⁴ independently represent H, Cl or Br, an alkyl group or an alkoxy group, R⁵ and R⁶ independently represent H or an alkyl group or together with the carbon atom to which they are bound form a cyclopentane ring or a cyclohexane ring, and X¹, X², X³ and X⁴ independently represent H, Cl or Br, wherein at least one of X¹, X², X³ and X⁴ is Cl or Br, or mixtures thereof,
said process comprising:
(a) providing an aromatic amine of the formula (2) 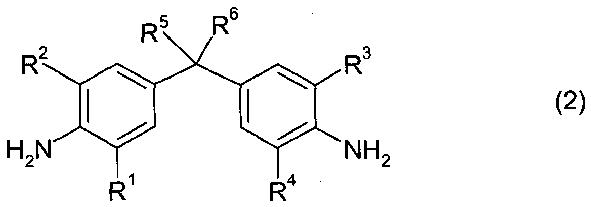 wherein R¹, R², R³, R⁴, R⁵ and R⁶ are as defined above, in the anilinium form in a mixture with sulfuric acid; and
(b) treating the mixture of step (a) with chlorine (Cl₂) or bromine (Br₂), followed by treatment with a base to obtain the halogenated aromatic amine of the formula (1).

2. The process of Claim 1, wherein R¹, R², R³ and R⁴ independently are an alkyl group and R⁵ and R⁶ are H or together with the carbon atom to which they are bound form a cyclohexane ring.

3. The process of Claim 1 or Claim 2, wherein X¹, X², X³ and X⁴ independently represent H or Cl.

4. The process of any one of Claims 1 to 3, wherein the reaction is carried out in a closed system.

5. The process of any one of Claims 1 to 3, wherein the reaction is carried out in an open system.

6. The process of any one of Claims 1 to 5, wherein the sulfuric acid has a sulfuric acid concentration of at least 85% by weight, preferably at least 95% by weight.

## Patentansprüche

1. Verfahren zur Herstellung von halogenierten aromatischen Aminen der Formel (1) 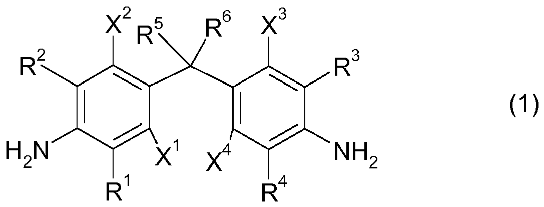 worin R¹, R², R³ und R⁴ unabhängig voneinander für H, Cl oder Br, eine Alkylgruppe oder eine Alkoxygruppe stehen, R⁵ und R⁶ unabhängig voneinander H oder eine Alkylgruppe sind oder zusammen mit dem Kohlenstoffatom, an das sie gebunden sind, einen Cyclopentanring oder einen Cyclohexanring bilden, und X¹, X², X³ und X⁴ unabhängig voneinander H, Cl oder Br bedeuten, wobei wenigstens eines von X¹, X², X³ und X⁴ Cl oder Br ist, oder von Gemischen derselben,
welches Verfahren umfasst:
(a) die Zurverfügungstellung eines aromatischen Amins der Formel (2) 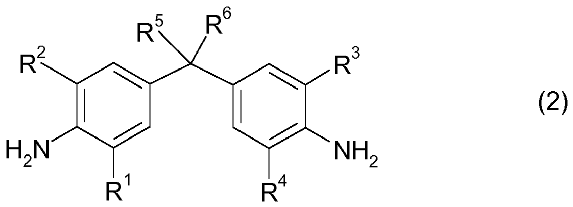 worin R¹, R², R³, R⁴, R⁵ und R⁶ wie oben definiert sind, in der Aniliniumform im Gemisch mit Schwefelsäure; und
(b) das Behandeln des Gemisches aus Schritt (a) mit Chlor (Cl₂) oder Brom (Br₂), gefolgt von einer Behandlung mit einer Base, um das halogenierte aromatische Amin der Formel (1) zu erhalten.

2. Verfahren nach Anspruch 1, worin R¹, R², R³ und R⁴ unabhängig voneinander eine Alkylgruppe sind und R⁵ und R⁶ H sind oder zusammen mit dem Kohlenstoffatom, an das sie gebunden sind, einen Cyclohexanring bilden.

3. Verfahren nach Anspruch 1 oder 2, worin X¹, X², X³ und X⁴ unabhängig voneinander H oder Cl bedeuten.

4. Verfahren nach irgendeinem der Ansprüche 1 bis 3, worin die Reaktion in einem geschlossenen System durchgeführt wird.

5. Verfahren nach irgendeinem der Ansprüche 1 bis 3, worin die Reaktion in einem offenen System durchgeführt wird.

6. Verfahren nach irgendeinem der Ansprüche 1 bis 5, worin die Schwefelsäure eine Schwefelsäurekonzentration von wenigstens 85 Gew.-%, vorzugsweise wenigstens 95 Gew.-% besitzt.

## Revendications

1. Procédé de préparation d'amines aromatiques halogénées de la formule (1) 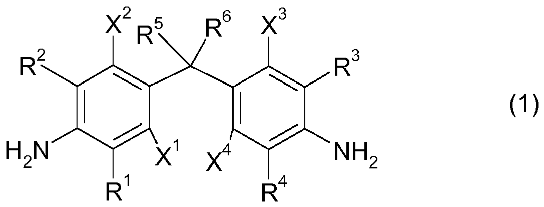 dans laquelle R¹, R², R³ et R⁴ représentent indépendamment H, Cl ou Br, un groupe alkyle ou un groupe alcoxy, R⁵ et R⁶ représentent indépendamment H ou un groupe alkyle ou, conjointement avec l'atome de carbone auquel ils sont liés, forment un noyau cyclopentane ou un noyau cyclohexane, et X¹, X², X³ et X⁴ représentent indépendamment H, Cl ou Br, au moins un de X¹, X², X³ et X⁴ représentant Cl ou Br, ou des mélanges de ceux-ci,
ledit procédé comprenant :
(a) l'obtention d'une amine aromatique de la formule (2) 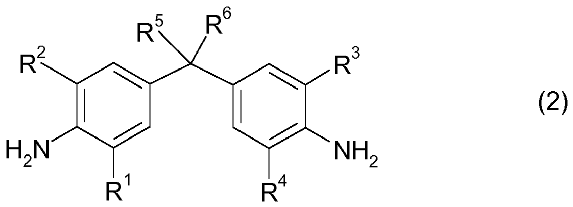 dans laquelle R¹, R², R³, R⁴, R⁵ et R⁶ sont tels que définis ci-dessus, sous la forme anilinium dans un mélange avec de l'acide sulfurique ; et
(b) le traitement du mélange de l'étape (a) avec du chlore (Cl₂) ou du brome (Br₂), suivi d'un traitement avec une base pour obtenir l'amine aromatique halogénée de la formule (1).

2. Procédé selon la revendication 1, dans lequel R¹, R², R³ et R⁴ représentent indépendamment un groupe alkyle et R⁵ et R⁶ représentent H ou, conjointement avec l'atome de carbone auquel ils sont liés, forment un noyau cyclohexane.

3. Procédé selon la revendication 1 ou la revendication 2, dans lequel X¹, X², X³ et X⁴ représentent indépendamment H ou CI.

4. Procédé selon l'une quelconque des revendications 1 à 3, dans lequel la réaction est réalisée dans un système fermé.

5. Procédé selon l'une quelconque des revendications 1 à 3, dans lequel la réaction est réalisée dans un système ouvert.

6. Procédé selon l'une quelconque des revendications 1 à 5, dans lequel l'acide sulfurique a une concentration d'acide sulfurique d'au moins 85 % en poids, de préférence au moins 95 % en poids.
